Europäisches Patentamt

European Patent Office

Office européen des brevets

(11) Publication number: **0 446 763 A2**

# EUROPEAN PATENT APPLICATION

(21) Application number: **91103306.6**

(22) Date of filing: **05.03.91**

(51) Int. Cl.5: **A61K 39/395, C12P 21/08**

(30) Priority: **06.03.90 JP 52825/90**

(43) Date of publication of application:
**18.09.91 Bulletin 91/38**

(84) Designated Contracting States:
**DE FR GB**

(71) Applicant: **The Calpis Food Industry Co., Ltd.**
**20-3, 2-chome Ebisu-Nishi**
**Shibuya-ku Tokyo(JP)**

(72) Inventor: **Ikuta, Kazuyoshi**
**2-38, Nishi 9-chome, Minami 16-jo, Chuo-ku**
**Sapporo-shi, Hokkaido(JP)**
Inventor: **Ohki, Kohji**
**Ra Bahn No. 101, 9-1-27, Shinkawa 4-jo,**
**Kita-ku**
**Sapporo-shi, Hokkaido(JP)**
Inventor: **Kimura, Takuro**
**9-4-12, Sumikawa 6-jo, Minami-ku**
**Sapporo-shi, Hokkaido(JP)**
Inventor: **Ohmura, Kazutaka**
**3-3-14, Kitakashiwa**
**Kashiwa-shi, Chiba-ken(JP)**

(74) Representative: **Kraus, Walter, Dr. et al**
**Patentanwälte Kraus, Weisert & Partner**
**Thomas-Wimmer-Ring 15**
**W-8000 München 22(DE)**

(54) Antigen for producing anti-idiotype antibody, anti-idiotype antibody and method for producing the anti-idiotype antibody.

(57) A antigen for producing an anti-idiotype antibody has an anti-HIV action. The antigen consists of an antibody reactable with a peptide consisting of an amino aced chain of 70th to 132nd, 68th to 94th or 84th to 104th amino acids as counted from the N-terminal of the human CD4 molecule. Anti-idiotype antibodies having an anti-HIV action are produced from the above antigens, respectively.

EP 0 446 763 A2

## BACKGROUND OF THE INVENTION

This invention relates to an antigen for producing an anti-idiotype antibody, that is an antibody produced by using, as an immunogen, peptide fragments of human CD4 protein, that is a peptide consisting of an amino acid chain of 70th to 132nd amino acids, a peptide consisting of an amino acid chain of 68th to 94th amino acids or a peptide consisting of an amino acid chain of 86th to 104th amino acids, each counted from the N-terminal of the CD4 molecule (referred to hereinafter as a human CD4 (70-132) peptide, a human CD4 (68-94) peptide and a human CD4 (86-104) peptide, respectively). The aforementioned antibody is reactable with the human CD4 (70-132) peptide, the human CD4 (68-94) peptide or the human CD4 (86-104) peptide and is referred to as an anti-CD4 (70-132) peptide antibody, an anti-CD4 (68-94) peptide antibody or an anti-CD4 (86-104) peptide antibody, respectively. This invention also relates to the above mentioned anti-idiotype antibody produced by using the antibody as an immunogen, and a method for producing the anti-idiotype antibody.

The human CD4 protein is a membrane protein constituting a cell membrane of the T-lymphocyte. Infection of the T-lymphocyte by the human immunodeficiency virus or HIV, the virus known to cause acquired human immunodeficiency syndromes (AIDS), is initiated with the binding of the HIV envelope protein with the CD4 protein as the first step. That is, the CD4 protein becomes a receptor protein of the cell in the course of HIV infection (as reported in Dalgleish et al., Nature, 312, 763-767 (1984), Klatzmann et al., Nature, 312, 767-768 (1984), Mc. Dougal et al., Sciona, 231, 382-385 (1986), Sodroski et al., Nature, 322, 470-474 (1986), Lasky et al., Cell, 50, 975-985 (1987)). Thus, the CD4 protein is believed to possess a site or domain specifically bound with the HIV envelope protein.

There have been proposed various methods for inhibiting the HIV activity by employing substances exhibiting anti-HIV activities. Specifically, there are known a method employing the soluble CD4 protein, that is a soluble protein formed by producing only the outer membrane region of the CD4 molecule, a method employing an anti-CD4 antibody, including a mouse monoclonal antibody prepared by using the human CD4 protein as the immunogen, such as, for example, OKT4, OKT4A (B, C, D, E and F), Leu 3a or MT 151 (Sattentau et al., Science, 234, 1120-1123 (1986)), (Jameson et al., Science, 240, 1335-1339 (1988)), and a method employing a fragmentary peptide of the CD4 protein capable of binding with the HIV envelope protein, such as, for example, CD4 (76-94), $Cys^{86}$-Bzl peptide (Lifson et al., Science, 241, 712-716 (1988)) or CD4 (70-132), $Cys^{86}$ or $Cys^{132}$-diAcm peptide (Hayashi et al.). With the above mentioned first method, the soluble CD4 protein is selectively bound with and thereby masks the HIV envelope protein to inhibit the binding of the CD4 protein on the cell with the HIV envelope protein. With the second method, the anti-CD4 antibody is selectively bound with and thereby masks the CD4 protein on the T-lymphatic cell to inhibit the binding of the CD4 protein with the HIV envelope protein. Finally, with the third method, the fragmentary peptide of the CD4 protein capable of binding with the HIV envelope protein is selectively bound with and thereby masks the HIV envelope protein to inhibit the binding of the CD4 protein on the T-lymphatic cell with the HIV envelope protein.

However, with the first method, employing the soluble CD4 protein, since the haft-life period in the blood of the soluble CD4 protein is so short that it needs to be administered to a living body in large dosage in short time intervals with increased expense and damages to health of the living body. Moreover, if the CD4 protein concentration in the blood is raised, the function proper to the CD4 protein, that is the funcion of recognizing an antigen, is impeded with the resulting lowering of the immunological function of the living body. With the second method of employing the anti-human CD4 antibody, the anti-human CD4 antibody known to date is not bound with the specific binding site with the HIV envelope protein on the CD4 protein such that the epitope peptide of OKT 4A and Leu 3a, that is the human CD4 peptides (11-40), (18-51), (18-69) or (26-47), are destitute of anti-HIV activities. On the other hand, the antibody molecule IgG, with the molecular weight of ca. 150kd, is much larger than the CD4 protein with the molecular weight of ca. 55kd, so that the CD4 protein is spatially covered to a large extent by the antibody IgG when the antibody IgG is bound to the CD4 protein. Thus, it may be contemplated that the anti-HIV action could be displayed even if the specific binding site on the CD4 protein with the HIV envelope protein is not actually masked. Accordingly, the anti-HIV action would not be displayed, if the anti-idiotype antibody were produced from such anti-human CD4 antibody. In addition, the CD4 protein on the T-lymphatic cell is masked by the anti-human CD4 antibody when administered to the human body, so that the function proper to the CD4 protein may be obstructed, similarly as with the soluble CD4 protein. Finally, with the third method of employing the fragmentary peptide capable of binding with the HIV envelope protein, the peptide is so low in molecular weight that the peptide exhibits only poor antigenecity when administered to the human body. Although the function proper to the CD4 protein is obstructed to a less extent, it is necessary to apply the peptide in higher concentration and in larger quantities on account of the shorter half-life period in the blood.

## SUMMARY OF THE INVENTION

It is a principal object of the present invention to provide an anti-idiotype antibody which does not obstruct the function proper to the CD4 protein and which is specifically bound with the HIV envelope protein to inhibit the binding thereof with the CD4 protein on the T-lymphatic cell to thereby display the anti-HIV action, a method for producing the anti-idiotype antibody, and an antigen employed for preparing the anti-idiotype antibody.

It is another object of the present invention to provide an anti-idiotype antibody which has a long half-life period in the blood and which is specifically bound directly with the HIV envelope protein, a method for producing the anti-idiotype antibody, and an antigen employed for producing the anti-idiotype antibody.

It is a further object of the present invention to provide an anti-idiotype antibody capable of inhibiting infection of T-lymphocytes by HIV and inactivating the T-lymphocytes infected by HIV to prevent contagion from an infected T-lymphocytes to an uninfected T-lymphocytes, a method for producing the anti-idiotype antibody and an antigen employed for producing the anti-idiotype antibody.

The above and other objects of the present invention will become more apparent from the following description.

In accordance with the present invention, there is provided an antigen for producing an anti-idiotype antibody having an anti-HIV action, the antigen consisting of an antibody reactable with a peptide consisting of an amino acid chain of 70th to 132nd, 68th to 94th or 84th to 104th amino acids as counted from the N-terminal of the human CD4 molecule.

In accordance with the present invention, there is also provided an anti-idiotype antibody having an anti-HIV action, the anti-idiotype antibody being produced from an antigen consisting of an antibody reactable with a peptide consisting of an amino acid chain of 70th to 132nd, 68th to 94th or 86th to 104th amino acids as counted from the N-terminal of the human CD4 molecule.

In accordance with the present invention, there is also provided a method for producing an anti-idiotype antibody having an anti-HIV action comprising producing the anti-idiotype antibody while using, as an immunogen, an antibody reactable with a peptide consisting of an amino acid chain of 70th to 132nd, 68th to 94th or 86th to 104th amino acids as counted from the N-terminal of the human CD4 molecule.

## DESCRIPTION OF THE PREFERRED EMBODIMENTS

The present invention will be explained in more detail hereinbelow.

The anti-idiotype antibody of the present invention is produced by employing as an antigen an antibody reactable with the human CD4 (70-132) peptide, the human CD4 (68-94) peptide or the human CD4 (86-104) peptide. The anti-idiotype antibody of the present invention as well as the antigen will be explained in connection with the method for preparation thereof.

As a first step, the human CD4 (70-132) peptide, the human CD4 (68-94) peptide and the human CD4 (86-104) peptide, which are employed as antigens for producing the anti-CD4 (70-132) peptide antibody, the anti-CD4 (68-94) peptide antibody and the anti-CD4 (86-104) peptide antibody, are prepared. Incidentally, while the human CD4 (68-94) peptide is not included in the human CD4 (70-132) peptide, the former peptide is easily synthesized so that it is conveniently employed. Any known methods for preparing the peptide may be employed. For example, the peptide may be prepared at high purity by solid phase synthesis, employing Fmoc amino acid (Sheppard et al., J. Chem. Soc., Chem. Comm., 165-166 (1985)). The amino acid sequences are determined on the basis of the Maddon's report (Maddon et al., Cell, 47, 333-348 (1986)). In more detail, an Fmoc amino acid pentafluorophenyl (Pfp) ester, corresponding to the C-terminal of each partial peptide, is attached to a p-alkoxybenzyl alcohol resin in dimethylformaldehyde in the presence of 4-dimethylamino pyridine. Then, another Fmoc amino acid Pfp ester to be attached next is attached by the condensation reaction to the firstly mentioned Fmoc amino acid Pfp ester.

It is noted that threonine and serine are introduced by using 1-oxo-2-hydroxy-dihydro-benzotriazine (DFBT) ester. After termination of the coupling reaction of the amino acids, the resin with the peptide attached thereto is treated with a 20 wt%-solution of piperidine in dimethylformamide to remove the N-terminal Fmoc group. The resulting product is acted upon by TFA-thioanisole at room temperature in the presence of m-cresol for de-protection and recovery of the object partial peptide from the resin. The peptide containing arginine residues is treated with trimethyl silyl bromide (TMSBr) - thioanisole / TFA.

The method for preparing the partial peptide of the CD4 protein is not limited to the above described method. For example, the method of chemical synthesis employing t-Boc amino acid (Merrifield, J. Am.

Chem. Soc., 85, 2149 (1963)) may be employed. Alternatively, it is possible to employ the method of producing a DNA corresponding to the partial peptide and inserting the DNA into a suitable vector for reproduction in animal cells or microorganisms to produce the object partial peptide.

Then, as a second step, using the so-produced peptide itself or the peptide carried on a suitable carrier protein, such as bovine serum albumin or hemocyanin, as an antigen, a mammal such as mouse or rat is immunized to produce the anti-CD4 (70-132) peptide antibody, the anti-CD4 (68-94) peptide antibody or the anti-CD4 (86-104) peptide antibody.

For immunization, any conventional method may be employed. For example, if a mouse is used, preferably the antigen is administered in an amount of 0.01 to 0.5 mg each time for each mouse having 5 to 10 weeks of age. If necesary, the antigen may be thoroughly mixed before administration thereof with a suitable adjuvant, such as complete Freund's adjuvant, incomplete Freund's adjuvant or alum adjuvant, and injected hypodermally, intravenously or intraperitoneally. The antigen is then administered at an interval of 1 to 3 weeks once or up to five times to effect thorough immunization. For immunization, the antigen may be applied directly to immuno-related organs, such as spleen, or to immuno-related cells, such as antigen-presenting cell, macrophage, T-lymphocyte or B-lymphocyte.

As the produced anti-CD4 (70-132) peptide antibody, anti-CD4 (68-94) peptide antibody or anti-CD4 (86-104) peptide antibody, it is possible to use a polyclonal antibody obtained in the blood serum in a superatant of a cell culture medium. It is, however, possible to prepare a monoclonal antibody to prepare an antibody capable of recognizing the uniform epitope on the human CD4 (70-132) peptide, the human CD4 (68-94) peptide or the human CD4 (86-104) peptide.

For preparing the monoclonal antibody, lymphocytes are sampled from the thus immunized animal in two to four days after the last immunizing operation. In preparing the lymphocytes, spleen, lymphoid nodule or peripheral blood may be employed. These lymphocytes are fused with myeloma cells.

As the myeloma cells, cells devoid of specific enzymes, such as, for example, mouse myeloma P3-X63-Ag8-U1(P3-U1), P3-X63-Ag8-653(X63-653), P3-NS1-1-Ag4-1(NS-1), or rat myeloma 210-RCY3-Ag123, are preferred. Cell fusion may be performed in accordance with the Koehler and Milstein's method (Nature, 256, 495 - 497 (1975)).

After the termination of the cell fusion, hybridomas are selected by using a selective culture medium, such as a HAT (hypoxanthine-aminopterin-thymidine) culture medium.

The hybridoma producing an antibody against the human CD4 peptide is selected by the enzyme immunoassay as later described and cloning by the limiting dilution method is repeated two or three times to produce the hybridoma cells producing the monoclonal antibody.

For investigating into which of the 63 amino acid residues of the human CD4 peptide (70-132) are recognized by the produced hybridomas, the human CD4 peptide (70-132) is roughly divided into three regions and the hybridomas are classified in accordance with the reactivity of the hybridomas with these regions. More specifically, on retrieval by enzyme immunoassay using the peptide (68-94), the peptide (86-104) and the peptide (70-132) as the antigens, there are obtained, besides the clonal strain reactable with the peptide (70-132) (hybridoma CP-9 deposited as FERM P-10864, BP-3011), the clonal strain reactable with the peptide (68-94) (hybridoma CP-1 deposited as FERM P-10862, BP-3009) and the clonal strain reactable with the peptide (86-104) (hybridoma CP-2, deposited as FERM P-10863, BP-3010).

The object monoclonal antibody may be obtained by inoculating the monoclonal antibody producing hybridoma cell strains thus produced in the abdominal cavity of the animal of the same genus and collecting the abdominal dropsy after multiplication, or by cultivating the cell strains in a cultivator.

The produced polyclonal and monoclonal antibodies may be used after purification as the occasion may demand. Purification may be performed by any known methods usually employed for protein, such as, for example, ammonium sulfate fractionation, ion exchanging, gel filtration or affinity chromatography.

Alternatively, an in vitro immunization method may be used to produce the above mentioned anti-CD4 (70-132) peptide antibody, the anti-CD4 (68-94) peptide antibody or the anti-CD4 (86-132) peptide antibody. When the in vitro immunization method is employed, lymphatic cell fractions are prepared from the peripheral blood of normal animals by the specific gravity centrifugal method by "PERCOLL" (produced by Pharmacia Fine Chemicals Co.), the red blood cell removal method by ammonium chloride or by the flow cytometry. The produced cell fractions are cultivated and the human CD4 (70-132) peptide, the human CD4 (68-94) peptide or the human CD4 (86-104) peptide is added to the culture liquid at a concentration of 0.001 to 1 mg/ml to cause phagocytosis of macrophages in the fractions to thereby cause antigen presentation. The co-existing helper T-cells are activated by the presented antigen to transfer the antigen information to the B-cells to produce the B-cells sensitized by the antigen, that is the B-cells producing the anti-CD4 (70-132) peptide antibody, anti-CD4 (68-94) peptide antibody or the anti-CD4 (86-104) peptide antibody. These B-cells are changed into strains by EB (Epstein-Barr) viruses or by cell fusion with myeloma to obtain a sustainedly produced polyclonal antibody. The B-cells thus changed into strains may be cloned two or three times by the limiting dilution method to obtain the monoclonal antibody.

The direct reactivity of the monoclonal antibody produced by the above method with the CD4 protein present on the membrane of the CD4(+) cell, such as MOLT-4 or MT-4, may be checked by the western blotting method. For example, about $2 \times 10^7$ of the above cells are collected. Then, by processing with a hypotonic phosphate buffer including a protease inhibitor, such as phenylmethyl sulfonyl fluoride (PMSF), or by homogenization, the cells are destroyed, and a crude core fraction is precipitated by centrifugation at 1,000 rpm for 5 minutes to produce a supernatant, which is centrifuged at 6,500 rpm for 20 minutes to produce a crude membrane fraction. This crude membrane fraction is separated by the SDS-10 wt% polyacrylamide electrophoresis in accordance with Laemmli's method (Nature, 227, 680 (1970)). Western blotting is then performed in accordance with the Towbin et al.'s method (Proc. Natl. Acad. Sci. USA, 76, 4350 - 4354 (1979)). On coloration with a secondary antibody bound with peroxidase by using the above mentioned anti-CD4 (70-132) peptide antibody, the anti-CD4 (68-94) peptide antibody or the anti-CD4 (86-104) peptide antibody as the primary antibody, a band with specific coloration may be noticed at a zone corresponding to the molecular weight of 55,000 on the CD4 molecule. This band may also be detected with the anti-CD4 monoclonal antibody or OKT 4. It may be seen from the above that the antibody produced by using as the antigen the human CD4 (70-132) peptide, the human CD4 (68-94) peptide or the human CD4 (86-104) peptide may react with cell-derived CD4 protein.

Meanwhile, the above anti-CD4 (70-132) peptide antibody, the anti-CD4 (68-94) peptide antibody or the anti-CD4 (86-104) peptide antibody is bindable with the cell-derived CD4 molecule and also recognizes the specific binding site with the HIV envelope protein, so that it exhibits an anti-HIV action when applied to an infection model system between the HIV sensitive cell and HIV. If, for example, $1 \times 10^6$ of MT-4 cells and the antibody are mixed so that the ultimate density amounts to 1 $\mu$g/ml and reacted for thirty minutes and subsequently the peptide with high HIV titer value formed in an HIV liquid diluted stepwise, that is a culture liquid of MOLT-4 cells infected by HTLV-III or LAV-1 strains, are added to the reaction mixture, infection may be noticed in a control devoid of the antibody even when the HIV liquid of extremely low concentration is used, whereas infection may only be noticed with all of the test domains only when the HIV solution of a higher concentration is used. Thus, the anti-HIV action may be evaluated by comparing the upper limit of the dilution factor for which the infection by HIV occurs, that is the HIV titer. The lower the HIV titer, the stronger is the anti-HIV action exhibited by the antibody.

As a third step, an anti-idiotype antibody against the human CD4 (70-132) peptide, the human CD4 (68-94) peptide or the human CD4 (86-104) peptide is produced by using as an immunogen the anti-CD4 (70-132) peptide antibody, the anti- CD4 (68-94) peptide antibody or the anti-CD4 (86-104) peptide antibody, obtained in the above second step, and such an anti-idiotype antibody exhibiting the anti-HIV action is separated.

Although the polyclonal antibody may be used as the antibody used as the immunogen, it is more preferred to use the monoclonal antibody.

For immunization, the process employed in the above mentioned second step may be employed. The anti-CD4 peptide antibody, preferably the monoclonal antibody, is bound with a carrier protein, such as BSA, to produce an antigen for preparing the anti-idiotype antibody. Alternatively, the anti- CD4 peptide antibody may be used directly as the antigen. This antigen is inoculated to an animal of the same species as that used in preparing the antibody. When the antibody molecule is applied to the animal of the same species, only the idiotype site of the antibody exhibits antigenecity, such that cells producing the antibody against the idiotype, although a few in number, are produced. The subsequent process for preparing the anti-idiotype antibody may be similar to that used in the second step. If a mouse is used, for example, 0.1 to 1 mg per mouse may preferably be immunized four or five times at an interval of one to two weeks.

The possible presence of the anti-idiotype antibody may be determined by, for example, the enzyme immunoassay -2 according Example 3-(4) as will be explained subsequently. Although the anti-idiotype antibody may be produced from the polyclonal antibody, the monoclonal antibody with uniform epitope may preferably be used in a similar manner to that described above. The anti-HIV action of the anti-idiotype antibody may be determined by the infection model system employing the above mentioned HIV sensitive cells.

As an alternative method, the anti-idiotype antibody may also be produced in accordance with the in vitro sensitizing method of the second step, using the anti-CD4 peptide antibody obtained in the second step as an antigen. The anti-CD4 peptide antibody may also be bound with a carrier protein, such as BSA, so as to be used the antigen.

The antigen may be added in an amount of 0.001 to 1 mg/ml. The antigen may be changed into strains by infection with EB viruses or cell fusion with myeloma to produce a polyclonal antibody. It may also be cloned to produce a monoclonal antibody. The anti-idiotype antibody against each human CD4 peptide may also be produced by a method totally different from the above described method, that is, by using the B-lymphocytes of the peripheral blood of an animal infected by AIDS. In an immunological system of an animal infected by the AIDS, the major region of the HIV envelope protein is recognized as the antigen determinant. It may be contemplated that the anti-idiotype antibody against the human CD4 (70-132) peptide, the human CD4 (68-94) peptide or the human CD4 (86-104) peptide behaves similarly to the anti-HIV envelope protein antibody capable of reacting with the specific binding site on the HIV envelope protein with the CD4 protein. Consequently, if B-lymphocytes are produced by the peripheral blood of the animal infected by the AIDS and changed into strains by EB viruses and the so-produced strains are stimulated and sensitized by using a suitable secondary antigen, such as the anti-CD4 (70-132) peptide antibody, only the cells producing the antibody corresponding to the anti-idiotype antibody is stimulated by the antigen and multiplied. These multiplied cells may be cultivated to produce the anti-idiotype antibody. These cells may also be cloned to produce a monoclonal antibody.

The present inventors have prepared a variety of anti-idiotype antibodies, using the mouse anti-CD4 (70-132) peptide antibody, the mouse anti-CD4 (68-94) peptide antibody and the mouse anti-CD4 (86-104) peptide antibody as the antigens. The present inventors have also separated five anti-idiotype monoclonal antibody producing cells satisfying the primary screening by the immunoenzymatic method and the secondary screening by assessment of the anti-HIV action and deposited two typical strains as the ID-55 (FERM P-11157, BP-3165), ID-62 (FERM P-11158, BP-3166).

## EXAMPLES OF THE INVENTION

### Example 1

Production of Mouse anti-CD4 (70-132) Peptide Monoclonal Antibody

(1) Preparation of Immunized Mouse Spleen Cell

A BALB/c female mouse with five weeks of age, supplied from Charles Riber Japan Inc., was immunized by intraperitoneal injection of 200 $\mu$g of the human CD4 peptide (70-132) simultaneously with complete Freund's adjuvant. After two weeks, 200 $\mu$g of the same peptide, dissolved in PBS (phosphate-buffered saline), was administered intraperitoneally for additional immunization. Similar immunizing processes were repeated further twice followed by administering 500 $\mu$g of the same peptide. After three days, the spleen was taken out, disintegrated in Dulbecco's modification of eagle's medium (DMEM), suspended and washed to produce spleen cells for fusion.

(2) Preparation of Mouse Mieloma Cells

Mouse myeloma P3-X63-Ag8-U1 was cultivated in a DMEM containing 10% of FCS and the cells in the logarithmic growth phase were collected as the parent strains for fusion.

(3) Cell Fusion

2 X $10^8$ of the spllen cells obtained in (1) and (2) and 4 X $10^7$ of the mouse myeloma cells were mixed thoroughly and centrifuged into pellet. After discarding the supernatant, the pellet was disintegrated thoroughly and admixed gradually with 0.5 ml of 50% polyethylene glycol 1500 manufactured by Boehringer Mannheim Inc. over 1 to 2 minutes to effect cell fusion. Then, 10 ml of the DMEM was added under agitation dropwise over about 10 minutes to complete the cell fusion. 1 ml of FCS was added to the mass to terminate the fusion reaction. After removing the polyethylene glycol supernatant by centrifugation, the pellet was washed with the DMEM containing 10% of FCS and the supernatant was removed by centrifugation. The pellet was suspended in a HAT culture medium (DMEM culture medium containing 100 $\mu$M of polyxanthine, 0.4 $\mu$M of aminopterin, 16 $\mu$M of thymidine, 0.03 % L-glutamine and 15 % FCS) and poured onto a 96-well microtiter plate at a rate of 0.2 ml per well so that 5 X $10^5$ cells were accommodated in each well. Cultivation was performed in a carbonic gas cultivator at 37°C and the HAT culture medium was renewed every other day at a rate of 0.1 ml per well. Cultivation and growth were continued for 7 to 10 days to produce hybridoma colonies.

(4) Enzyme Immunoassay - 1

6

A 0.1 to 1 $\mu$g/ml solution of the human CD4 peptide dissolved in PBS was poured onto a 96-well microtiter plate for ELISA manufactured by Sumitomo Bakelite Co., Ltd. at a rate of 50 $\mu$l per well and allowed to stand at room temperature for 1 to 4 hours to allow the peptide to be adsorbed onto the bottom surface of each well. After washing three times with a phosphate buffer (PBS) containing 0.15 M of sodium chloride, with pH of 7.4, PBS containing 3% of casein sodium was charged into each well and allowed to stand for one hour at room temperature for coating the plate wells in their entirety with casein. After discarding the liquid in each well and washing with PBS, a supernatant liquid of the hybridoma culture was added at a rate of 50 $\mu$l per well and allowed to stand at room temperature for 1 to 4 hours. After washing five times with PBS containing 0.05% Tween 20 and washing twice with PBS, a solution of an anti-mouse IgG antibody bound with peroxidase (manufactured by Nippon Bio-Rad Laboratories), diluted to a concentration of 1/4,000 with PBS, was poured at a rate of 50 $\mu$l per well, and allowed to stand at room temperature for 1 to 2 hours. After washing five times with 0.05% Tween 20-PBS and twice with PBS, a coloring liquid prepared by mixing a 50 mM of a sodium acetate buffer containing 10 mM of orthophenylenediamine and 0.025% of BSA (pH 5.0) with 0.1% hydrogen peroxide at a ratio of 3 : 1 was added at a rate of 50 $\mu$l per well and allowed to stand at room temperature for 15 to 30 minutes for reaction. 1N sulfuric acid containing 0.1% sodium sulfite ($Na_2SO_3$) was added to the reaction system at a rate of 150 $\mu$l per well. After termination of the reaction, light absorbance was measured at a wavelength of 492 nm. In retrieving the object hybridoma, it suffices to select strains with a high value of measurement.

(5) Hybridoma Screening and Cloning

The supernatant culture liquid of each well was used as the antibody liquid and subjected to the above mentioned enzyme immunoassay - 1. The human CD4 (70-132) peptide was used as the antigen.

The hybridoma in the well which showed antibody production was cloned by the limiting dilution method. Specifically, the hybridoma was suspended in the HAT medium (HT medium) not containing aminopterin and adjusted to a cell concentration of 1 cell / 0.1 ml. Spleen cells prepared from the normal mouse were similarly suspended in the HT medium to a cell concentration of 5 X $10^5$ cells / 0.1 ml and the resulting suspension was poured on a 96-well microtiter plate at a rate of 0.1 ml per well. The above mentioned hybridoma suspension liquid was added at a rate of 0.1 ml per well. The plate containing one hybridoma cell in each well was cultivated in a carbonic acid gas cultivator at 37° C for 1 to 2 weeks.

An enzyme immunoassay was performed, by using the supernatant culture liquid contained in the well containing only one hybridoma colony as the antibody liquid, and the hybridoma exhibiting antibody positivity was again cloned by the limiting dilution method in the same way as mentioned hereinabove. Using the produced anti-CD4 (70 - 132) peptide antibody producing cells as the clonal strains, 11 independent hybridoma clonal strains CP1 to CP 11 were obtained and cultivated in large quantities in a 10% FCS-DMEM culture medium.

(6) Purification of Monoclonal Antibody

Each 50 ml of the 11 hybridoma strains produced in (5) above was cultivated in a culture medium free of serum ASF 103 manufactured by Ajinomoto Co., Ltd. The cultured products with the cell concentration of 1 X $10^6$ / ml were centrifuged and the produced supernatants were subjected to the protein A-agarose manufactured by Nippon Bio-Rad Laboratories or to the LCA lectin-cepharose manufactured by Pharmacia Fine Chemicals Co.. The supernatants subjected to the protein A-agarose were eluted by a citric acid buffer solution with pH of 4.0, while the supernatants subjected to the LCA lectin-cepharose were eluted by PBS containing 10% methyl-$\alpha$-D-mannopyranoside. The eluted products were dialyzed by PBS to produce purified monoclonal antibodies.

As an alternative method, hybridoma was cultivated in the mouse abdominal dropsy and anti-abdominal dropsy antibody was prepared and purified.

Each 1 X $10^7$ cells of the anti-CD4 (70-132) monoclonal producing cell strains CP1 to 11 were intraperitoneally injected to a BALB/c mouse, to which 0.5 ml of 2,6,10,14-tetramethyl-pentadecane manufactured by Wako Junyaku Co., Ltd. had been intraperitoneally administered two weeks before. After 5 to 10 days following the injection, the swollen abdomen of the mouse was incised and the liquid in the abdominal cavity was recovered. After centrifugation at 2,000 rpm for 5 minutes, the supernatant liquid was admixed with an equal amount of PBS and mixed together. The resulting mixture was further admixed with an equal amount of a 100% saturated ammonium sulfate solution to produce a 50% saturated ammonium sulfate solution. After being allowed to stand at 4° C for one hour, the latter solution was centrifuged at 8,000 rpm at 4° C for 15 minutes and the precipitates mainly consisting of IgG were recovered. 6 ml of PBS was added to the precipitates and 4 ml of a 100% saturated ammonium sulfate solution was added to the resulting solution to give a 40% saturated ammonium sulfate solution. After being allowed to stand at 4°C for one hour, the latter was centrifuged at 8,000 rpm at 4°C for 15 minutes and the precipitates were recovered. The precipitates were dissolved in PBS and added to a column of "Protein G Sepharose" buffered by PBS. (manufactured by Pharmacia Fine Chemicals Co.)

The precipitates were washed with PBS having a volume as much as ten times the column capacity and eluted with 0.1 M of glycine-hydrochloric acid (pH 2.7). The eluated solution was received immediately in a 1M Tris-HCl buffer solution (pH 7.5) and the antibody fraction was recovered. The produced antibody fraction was dialyzed four times against 4 liters of PBS to produce the purified monoclonal antibodies.

(7) Reactivity of Monoclonal Antibody with CD4 Protein

The reactivity of the monoclonal antibodies obtained in (6) above was checked by using the western blotting method.

$2 \times 10^7$ of MOLT-4cells (CD-4+) were washed twice by centrifugation with PBS and admixed with 2 ml of a 10 mM sodium phosphate buffer containing 1 mM of PMSF (pH 7.5). The resulting mass was suspended and processed hypotonically under ice cooling for 5 minutes. 4 ml of PBS containing 1 mM PMSF was added and the resulting mixture was homogenized by a Dounce type homogenizer under ice cooling by 40 to 50 strokes. The homogenized liquid was centrifuged at 1,000 rpm for 5 minutes. The supernatant was again centrifuged at 1,000 rpm for 5 minutes and the so-produced supernatant was further centrifuged at 6,500 rpm for 20 minutes. The resulting precipitates were used as a crude membrane fraction in electrophresis. After separation by electrophoresis in a 10% polyacrylamide gel containing 0.1%SDS, the protein was electrically transfered from polyacrylamide gel to a nitrocellulose paper in a 25 mM trishydroxyaminomethane-192 mM glycine buffer solution containing 20% methanol (pH 8.3). The nitrocellulose paper was coated with 3% casein-PBS by immersing the paper therein at room temperature for one hour. The coated paper was reacted at 4°C overnight with the monoclonal antibodies, washed, and reacted for about two hours with a 1,000 -fold diluted solution of the anti-mouse IgG (H + L) antibody labeled with peroxidase (manufactured by Cappel AG). The reaction mass was washed and reacted with a peroxidase substrate liquid containing 0.5 mg/ml of 4-chloro-1-naphthol (manufactured by Seikagaku Kogyo Co., Ltd.) and 0.02% of hydrogen peroxide at room temperature for 30 minutes and the band of protein coloration was checked.

The 11 monoclonal antibodies, obtained in (6) above, were specifically reacted only with the protein of the human CD4 molecule having the molecular weight of 55,000.

Example 2

Anti-HIV Effect of Anti-CD4 (70-132) Peptide Monoclonal Antibody

50 μl of $1 \times 10^6$ HIV sensitive MT4 cells (CD4+) per ml and 50 μl of each of monoclonal antibodies (CP1-11) at 2 μg/ml were mixed together on a 96-well microtiter plate and allowed to stand at room temperature for 30 minutes. To each of the mixtures was added and mixed 100 μl of the HIV liquid diluted stepwise by a factor of $10^1$ to $10^5$. As the HIV liquids, the supernatants of the MOLT-4 cell culture liquids sustainedly infected by HIV-1 were employed. Each of the resulting mixtures was cultivated at 37°C for four days in a carbonic acid gas cultivator on an RPMI-1640 culture medium containing 10% FCS.

Infection was determined by the presence or absence of the gag protein p18 of HIV reproduced in a specimen which was obtained by applying and securing the above cells on a slide glass by -20°C acetone. The protein p18 was detected by the immunological fluorescent antibody technique employing the anti-p18 monoclonal antibody.

In accordance with the immunological fluorescent antibody technique, the anti-p18 antibody solution was applied dropwise on the cells secured on the slide glass, reacted at room temperature for 15 minutes and washed with PBS. The anti-mouse IgG antibody solution coupled with "Fluorescein", manufactured by Cappel AG, was diluted by PBS by a factor of one-fortieth and applied dropwise to the raction system for reaction at room temperature for 15 minutes. The reaction product was washed with PBS and, after sealing with 90% glycerin, the possible presence of the specific green to yellow fluorescent coloration was checked by a fluorescence microscope.

The anti-HIV effect was determined by comparison of the upper limits of the HIV liquid dilution factor before the actual infection (HIV titers). That is, the lower the HIV titer than the control, the stronger is the anti-HIV effect.

In all of the anti-CD4 (70-132) monoclonal antibodies, the anti-HIV effect was seen to lower the HIV titer by a factor of $10^1$ to $10^3$ as compared to the control.

Above all, the strong anti-HIV effect was noticed with CP-1, CP-2 and CP-9.

Example 3

Preparation of Anti-idiotype Monoclonal Antibodies

(1) Preparation of Antigen

An anti-CD4 (70-132) monoclonal antibody was prepared in accordance with Example 1-(6) and purified so as to be used as the immunogen. Separately, an anti-CD4 (68-94) monoclonal antibody and an anti-CD4 (86-104) monoclonal antibody was prepared similarly in accordance with Example 1-(6), respectively.

(2) Method of Immunization and Spleen Cells

A solution in 0.5 ml of a PBS solution of 1 mg of purified anti-CD4 (70-132) peptide monoclonal antibody was mixed thoroughly with 0.5 ml of the complete Freund's adjuvant and administered to the abdominal cavity of a BALB/c mouse. A solution which was the same as the above solution free of the adjuvant was administered thrice at an interval of one week into the abdominal cavity of the same mouse. The spleen was taken out three days after the last immunizing operation. Subsequently, preparation of the spleen cells and myeloma and cell fusion were carried out in accordance with the method of preparing the monoclonal antibody as explained in Example 1.

The same procedure was followed with respect to the anti-CD4 (68-94) monoclonal antibody and the anti-CD4 (86-104) monoclonal antibody.

(3) Enzyme Immunoassay - 2

An anti-mouse IgG(H + L) antibody (manufactures by Cappel AG), diluted by PBS at 1 µg/ml, was poured onto a 96-well microtiter plate for ELISA manufactured by Coster Inc. at a rate of 50 µl per well and allowed to stand at room temperature for 1 to 4 hours to allow the antibody to be adsorbed to the bottom surface of the well. After washing three times with PBS, each well was filled with a phosphate buffer (PBS) containing 3% casein sodium. The microtiter plate was allowed to stand at room temperature for one hour for coating the plate wells in their entirety with caein. After discarding the liquid in each well and washing with PBS, a supernatant liquid of the cultured hybridoma was added at a rate of 50 µl per well and allowed to stand at room temperarture for 1 to 4 hours. After washing five times with PBS containing 0.05% Tween 20, a 1 µg/ml PBS solution of an antibody obtained by biotinating an anti-CD4 (70-132) monoclonal antibody used as an antigen, with the use of a biotinating agent manufactured by Pierce Inc., was added at a rate of 50 µl per well, and allowed to stand at room temperature for 1 to 2 hours. Avidin bound with horseradish peroxidase, manufactured by Vector Inc. diluted by a dilution factor of 2,000 by PBS, was added at a rate of 50 µl per well and the resulting product was allowed to stand at room temperature for 1 to 2 hours. After washing five times with PBS containing 0.05% Tween 20, coloration and measurement were performed with the use of orthophenylene diamine in the same way as in Example 1-(4). For retrieving the object hybridoma, it suffices to select the cultured supernatant liquid having the high value of measurement.

The same procedure was followed with respect to the anti-CD4 (68-94) monoclonal antibody and the anti-CD4 (86-104) monoclonal antibody.

(4) Hybridoma Screening and Cloning

The operation is similar to that of Example 1-(5). The anti-idiotype antibody producing hybridomas obtained in the above described manner was cloned by repeating the limiting dilution method twice. The cloned hybridomas were selected in accordance with the above mentioned enzyme immunoassay - 2 to produce 24 clones manufacturing positive antibodies, respectively. These clones were cultivated in large quantities on the 10% FCS - DMEM.

Example 4

Anti-HIV Effect of Anti-idiotype Antibodies

Similarly to Example 2, the antibodies each obtained in Example 3 were purified in accordance with the method shown in Example 1-(6) and diluted at a ratio of 3 $\mu$g/ml with RPMI-1640 medium. 50 $\mu$l of each of the diluted antibodies and 100 $\mu$l of the HIV solution diluted stepwise by dilution factors of $10^1$ to $10^6$ were mixed on a 96-well microtiter plate and allowed to stand at room temperature for 30 minutes. 50 $\mu$l of a solution of MT-4 cells at 2 X $10^6$ cells/ml were added to each of the resulting mixtures and cultivated on a RPMI-1640 medium containing 10% of FCS in carbonic acid gas cultivators at 37°C for four days, respectively.

Similarly to Exmple 2, infection was determined in accordance with the immuno-fluorescence antibody technique and the anti-HIV effect was determined by comparison of the HIV titers.

As shown in Table 1, a strong anti-HIV action was exhibited with ID-38, ID-51, ID-55 and ID-62 in which the HIV titer was decreased by a factor of $10^3$.

## Table 1

### Anti-HIV Effect of Anti-Idiotype Monoclonal Antibodies

| Hibridoma used for Producing Monoclonal Antibody as Antigen | Anti-idiotype Antibody | HIV Titer |
|---|---|---|
| | control (-) | $10^{4.5}$ |
| CP9 | ID-38 | $10^{1.5}$ |
| CP9 | ID-50 | $10^{2.5}$ |
| CP2 | ID-51 | $10^{1.5}$ |
| CP9 | ID-55 | $10^{1.5}$ |
| CP1 | ID-62 | $10^{1.5}$ |

The amino acid sequence of the human CD4 peptides in the present invention is as follows:

Peptide (70-132)

Pro-Leu-Ile-Ile-Lys-Asn-Leu-Lys-Ile-Glu-Asp-Ser-

Asp-Thr-Tyr-Ile-Cys-Glu-Val-Glu-Asp-Gln-Lys-Glu-Glu-Val-

Gln-Leu-Leu-Val-Phe-Gly-Leu-Thr-Ala-Asn-Ser-Asp-Thr-His-Leu-

Leu-Gln-Gly-Gln-Ser-Leu-Thr-Leu-Thr-Leu-Glu-Ser-Pro-Pro-Gly-

Ser-Ser-Pro-Ser-Val-Gln-Cys

Peptide (68-94)

Asn Phe Pro Leu Ile Ile Lys Asn Leu Lys Ile Glu
          70                 75

Asp Ser Asp Thr Tyr Ile Cys Glu Val Glu Asp Gln Lys Glu Glu
 80               85              90

Peptide (86-104)

Cys Glu Val Glu Asp Gln Lys Glu Glu Val Gln Leu
                90              95

Leu Val Phe Gly Leu Thr Ala
    100

Applicant: The Calpis Food Industry

## Sequence Listing

Sequence identification number: (1) to (3)

Sequence type: proteins

Sequence length: (1) 63 amino acids

(2) 27 amino acids

(3) 19 amino acids

Original source: human CD4 protein

Features: (1) Peptide 70 to 132 of CD4

(2) Peptide 68 to 94 of CD4

(3) Peptide 86 to 104 of CD4

Properties: Binding with HIV gp 120

(1) Peptide (70 - 132)

```
        Pro Leu Ile Ile Lys Asn Leu Lys Ile Glu
        70                  75

Asp Ser Asp Thr Tyr Ile Cys Glu Val Glu Asp Gln Lys Glu
  80              85                  90

Glu Val Gln Leu Leu Val Phe Gly Leu Thr Ala Asn Ser Asp
      95              100                 105

Thr His Leu Leu Gln Gly Gln Ser Leu Thr Leu Thr Leu Glu
      110             115                 120

Ser Pro Pro Gly Ser Ser Pro Ser Val Gln Cys
          125             130
```

(2) Peptide (68 - 94)

```
        Asn Phe Pro Leu Ile Ile Lys Asn Leu Lys
            70                  75

Ile Glu Asp Ser Asp Thr Tyr Ile Cys Glu Val Glu Asp Gln
      80              85                  90

Lys Glu Glu
```

(3) Peptide (86 - 104)

```
        Cys Glu Val Glu Asp Gln Lys Glu Glu Val
                        90                  95

Gln Leu Leu Val Phe Gly Leu Thr Ala
          100
```

## Claims

1. An antigen for producing an anti-idiotype antibody having an anti-HIV action, said antigen consisting of an antibody reactable with a peptide consisting of an amino acid chain of 70th to 132nd amino acids as counted from N-terminal of human CD4 molecule.

2. An anti-idiotype antibody having an anti-HIV action, said anti-idiotype antibody being produced from the antigen according to claim 1.

3. A method for producing an anti-idiotype antibody having an anti-HIV action comprising producing the anti-idiotype antibody while using, as an immunogen, of an antibody reactable with a peptide consisting of an amino acid chain of 70th to 132nd amino acids as counted from N-terminal of human CD4 molecule.

4. An antigen for producing an anti-idiotype antibody having an anti-HIV action, said antigen consisting of an antibody reactable with a peptide consisting of an amino acid chain of 68th to 94th amino acids as counted from N-terminal of human CD4 molecule.

5. An anti-idiotype antibody having an anti-HIV action, said anti-idiotype antibody being produced from the antigen according to claim 4.

6. A method for producing an anti-idiotype antibody having an anti-HIV action comprising producing the anti-idiotype antibody while using, as an immunogen, an antibody reactable with a peptide consisting of an amino acid chain of 68th to 94th amino acids as counted from N-terminal of human CD4 molecule.

7. An antigen for producing an anti-idiotype antibody having an anti-HIV action, said antigen consisting of an antibody reactable with a peptide consisting of an amino acid chain of 86th to 104th amino acids as counted from N-terminal of human CD4 molecule.

8. An anti-idiotype antibody having an anti-HIV action, said anti-idiotype antibody being produced from the antigen according to claim 7.

9. A method for producing an anti-idiotype antibody having an anti-HIV action comprising producing the anti-idiotype antibody while using, as an immunogen of an antibody reactable with a peptide consisting of an amino acid chain of 86th to 104th amino acids as counted from N-terminal of human CD4 molecule.